(19) Europäisches Patentamt
European Patent Office
Office européen des brevets

(11) Numéro de publication: **0 204 049**
**A1**

(12) **DEMANDE DE BREVET EUROPEEN**

(21) Numéro de dépôt: **85401043.6**

(22) Date de dépôt: **28.05.85**

(51) Int. Cl.⁴: **C 07 D 217/00**

(43) Date de publication de la demande:
**10.12.86 Bulletin 86/50**

(84) Etats contractants désignés:
**AT BE CH DE GB IT LI LU NL SE**

(71) Demandeur: **SYNTHELABO**
**58, rue de la Glacière**
**F-75621 Paris Cedex 13(FR)**

(72) Inventeur: **Rossey, Guy**
**8, Square Lebrun Voisins-le-Bretonneux**
**F-78180 Montigny-le-Bretonneux(FR)**

(72) Inventeur: **Legroux, Didier**
**70 Domont Village**
**F-95330 Domont(FR)**

(74) Mandataire: **Thouret-Lemaitre, Elisabeth et al,**
**Service Brevets - SYNTHELABO 58, rue de la Glacière**
**F-75621 Paris Cedex 13(FR)**

(54) **Dérivés d'isoquinoléine, leur préparation et leurs applications.**

(57) Dérivés d'isoquinoléine répondant à la formule (I)

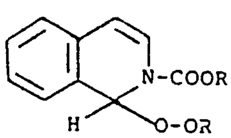
(I)

dans laquelle
R représente le radical tertiobutyle ou le radical

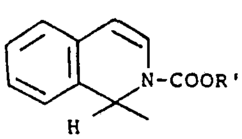

et
R' représente le radical $CH_3$ ou $C_2H_6$.
Application à la préparation de l'isoquinolone-1(2*H*).

EP 0 204 049 A1

La présente invention a pour objet des dérivés d'isoquinoléine, leur préparation et leurs applications.

Les composés de l'invention répondent à la formule suivante

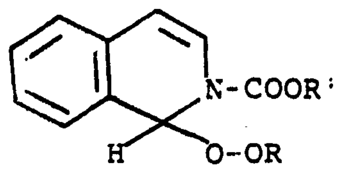

(I)

dans laquelle
R représente le radical tertiobutyle ou le radical

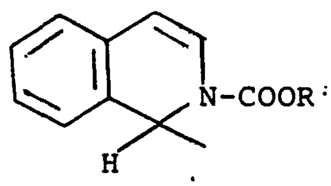

et
R' représente le radical $CH_3$ ou $C_2H_5$.

Selon l'invention on peut préparer les composés (I) à partir de l'isoquinoléine par réaction avec l'hydroperoxyde de tertiobutyle ou l'eau oxygénée, dans un solvant tel que le chlorure de méthylène, les alcools (méthanol, éthanol, propanol), en présence d'une base telle que la triéthylamine ou le bicarbonate de Na ou K et de chloroformiate de méthyle ou d'éthyle, à une température de 0°C à 30°C.

Les composés de l'invention peuvent être utilisés pour la préparation de l'isoquinolone-1(2<u>H</u>), composé utile pour la synthèse du composé 7 décrit dans le brevet français 81 10428 de la Demanderesse.

La conversion des composés (I) en isoquinolone-1 (2<u>H</u>) est effectuée en milieu basique dans un solvant tel que les solvants polaires aprotiques ou protiques, par exemple par traitement du composé (I) par de la soude à 50 % dans du

diméthylsulfoxyde ou par un mélange méthoxyde de sodium/méthanol.
Les exemples suivants illustrent l'invention.

Les analyses et les spectres IR et RMN confirment la structure des composés.

Exemple 1 (Diméthyl-1,1 éthyl) dioxy -1 isoquinoléine-2 (1*H*)-carboxylate de méthyle.

Dans un ballon de 100 ml, placé dans un bain de glace, muni d'une ampoule à pression constante et d'une agitation magnétique, on introduit 6,5 g d'isoquinoléine, 25 ml de $CH_2Cl_2$, 7,5 g de $Et_3N$ et 6,4 g de $Me_3COOH$ : puis on ajoute goutte à goutte, 5,8 ml de $ClCOOCH_3$ (légèrement exothermique) en 15 mn et on agite le milieu réactionnel pendant 2 heures.

On ajoute ensuite 100 ml d'eau, décante la phase organique, lave avec 20 ml d'eau. On décante et sèche sur $Na_2SO_4$, puis concentre : on obtient un résidu incolore qui se solidifie à froid.

Après recristallisation dans un mélange isopropanol/eau le composé fond à 72,7 - 73°C.

Exemple 2 Bis- (oxy-1 isoquinoléine-2(1*H*)-carboxylate de méthyle).

Dans un ballon de 100 ml maintenu à - 10°C, on introduit 12,9g d'isoquinoléine, 50 ml de chlorure de méthylène, 12,6 g de bicarbonate de sodium et 10 ml de $H_2O_2$.
On ajoute goutte à goutte 11,6 ml de $ClCOOCH_3$ en 15 minutes, puis laisse réagir pendant 2 heures pendant que la température remonte à la température ambiante.
On ajoute 50 ml d'eau au mélange réactionnel et 300 ml d'éther diisopropylique ; on filtre le solide blanc puis sèche sous vide à 25°C pendant une nuit.
On obtient le composé qui fond à 175°C.

0204049

Exemple 3 Isoquinolone - 1(2H)

Dans un ballon de 250 ml muni d'un réfrigérant, d'une agitation magnétique, on introduit 14,83 g du produit obtenu dans l'exemple 1, 100 ml de MeOH, puis 14 ml d'un mélange $MeO^-Na^+$/MeOH 29 %.

On porte à la température du reflux (bain à 93°C) pendant 65 mn.
Le milieu réactionnel jaunit légèrement puis s'opacifie.
On laisse refroidir, ajoute 20 ml d'$H_2O$ et concentre la solution, puis ajoute 100 ml d'$H_2O$. On filtre le solide formé et le met en suspension dans 30 ml de tBuOMe, filtre et sèche le composé à l'air puis sous vide. F = 212°C.

Exemple 4 : Isoquinolone-1(2H).

Dans un ballon de 100 ml, on introduit 2 g du composé obtenu dans l'exemple 2, 10 ml de DMSO, 8 g de $NaHCO_3$ et porte le mélange réactionnel à 110°C pendant 3 heures.
On ajoute alors 80 ml d'eau, laisse recristalliser l'isoquinolone que l'on filtre et sèche.
On la purifie en la mettant en suspension dans du tBuOMe et la filtrant comme dans l'exemple 3. F = 212°C.

Revendications pour les états contractants : BE, CH, DE, GB, IT, LI, LU, NL, SE.

1. Dérivés d'isoquinoléine répondant à la formule (I)

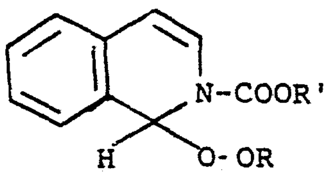

(I)

dans laquelle

R représente le radical tertiobutyle ou le radical

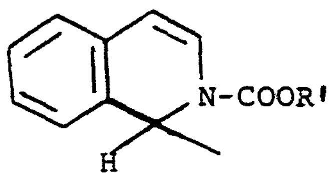

et

R' représente le radical $CH_3$ ou $C_2H_5$.

2. Procédé de préparation des composés selon la revendication 1, procédé caractérisé en ce que l'on fait réagir l'isoquinoléine avec de l'hydroperoxyde de tertiobutyle ou de l'eau oxygénée, en présence d'une base et d'un chloroformiate.

3. Application des composés selon la rvendication 1, à la préparation de l'isoquinolone-1(2H).

0204049

Revendication pour l'état contractant : AT.

Procédé de préparation de dérivés d'isoquinoléine répondant à la formule (I)

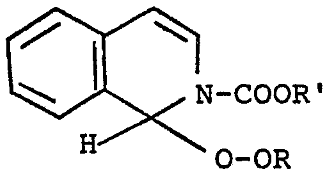

(I)

dans laquelle
R représente le radical tertiobutyle ou le radical

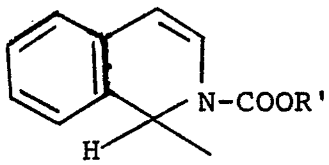

et
R' représente le radical $CH_3$ ou $C_2H_5$,
procédé caractérisé en ce que l'on fait réagir l'isoquinoléine avec de l'hydroperoxyde de tertiobutyle ou de l'eau oxygénée, en présence d'une base et d'un chloroformiate.

Office européen des brevets

**RAPPORT DE RECHERCHE EUROPEENNE**

0204049

Numéro de la demande

EP 85 40 1043

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| | Néant<br>----- | | C 07 D 217/00 |
| | | | DOMAINES TECHNIQUES RECHERCHES (Int. Cl.4) |
| | | | C 07 D 217/00 |

Le présent rapport de recherche a été établi pour toutes les revendications

| Lieu de la recherche | Date d'achèvement de la recherche | Examinateur |
|---|---|---|
| LA HAYE | 16-01-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82